# EUROPEAN PATENT APPLICATION

(11) **EP 4 249 009 A1**
(43) Date of publication of application: **27.09.2023**
(21) Application number: 21909450.5
(22) Date of filing: 22.12.2021
(51) Int. Cl.: A61L 27/28, A61L 27/34, A61L 27/36, A61L 27/50, C08G 65/44

(54) **LONG-ACTING SUPERHYDROPHOBIC ANTICOAGULATION BIOVALVE AND PREPARATION METHOD THEREFOR**

(30) Priority: 22.12.2020 CN 202011524829
(71) Applicant: Jilin Venus Haoyue Medical Limited, Changchun, Jilin 130000 (CN)
(72) Inventor: YANG, Li, Chengdu, Sichuan 610064 (CN); WANG, Yunbing, Chengdu, Sichuan 610064 (CN); LUO, Rifang, Chengdu, Sichuan 610064 (CN)
(74) Representative: KIPA AB
(86) International application number: PCT/CN2021/140378
(87) International publication number: WO 2022/135454

(57) **Abstract**

A long-acting super-hydrophobic anticoagulant biological valve and a preparation method therefor. The preparation method includes the following steps: (1) treating a biological valve material with glutaraldehyde; (2) placing the biological valve material treated in step (1) into an acid liquid containing a polyphenol compound and metal ions, and adding an oxidant for reaction; and (3) reacting the biological valve material treated in step (2) with a hydrophobic substance. According to the method, a super-hydrophobic coating having a long-acting high water contact angle and a low rolling angle is prepared on the surface of the biological valve by means of a simple and stable operation process without affecting the performance of the valve body, and the requirements of long-acting anticoagulation are met by resisting the adsorption of plasma proteins.

## Description

### TECHNICAL FIELD

The invention belongs to the technical field of valve material preparation, and in particular relates to a long-acting super-hydrophobic anticoagulant biological valve and a preparation method therefor.

### DESCRIPTION OF THE PRIOR ART

Along with the social structure of population in China aging, the number of patients with severe valvular stenosis and regurgitation has increased significantly than before. The overall incidence rate among people over 75 years old is relatively high. Once patients have symptoms and have not received treatment, close to 50% of them may die within 2 to 3 years. Minimally invasive interventional valve surgery is becoming an important treatment accepted by more and more patients because of its small surgical trauma, low risk and good effect. With the development of minimally invasive intervention technology, however, many problems occur. Among them, the implantation of biological valve in interventional therapy may lead to thrombosis, and the detachment of thrombus from the surface of the valve will increase the risk of postoperative stroke; and the thrombosis on the surface of the valve will seriously affect the durability of the valve. Therefore, a biological valve material having an excellent long-acting anticoagulant effect itself is very important for the durability of the valve and the safety of the patient's life.

A super-hydrophobic surface with a bionic structure of lotus leaf has been widely studied due to its high water contact angle (>150°) and low rolling angle, and is mainly used in the fields of self-cleaning, moisture resistance, water - oil separation, corrosion resistance and stain resistance. Once the surface of a common biomaterial comes into contact with blood or body fluid, a large number of proteins or cells will adhere to the surface of the material, which will seriously affect the performance of the material. However, superhydrophilic surfaces and super-hydrophobic surfaces both have a good stain resistance (resistance to non-specific adhesion of proteins, cells and microorganisms). Studies have reported that, compared with superhydrophilic surfaces, super-hydrophobic surfaces have lower platelet adhesion ability, indicating that super-hydrophobic surfaces have better antithrombotic properties. Currently, the super-hydrophobic surfaces are mainly prepared on the surface of metals, polymers and inorganic materials, and few studies are made on the preparation of super-hydrophobic coatings on the surface of biological valve materials. Therefore, the preparation of super-hydrophobic coatings with high water contact angle and low adhesion on the surface of biological valve materials are crucial to resist thrombosis after the valve being implanted in vivo.

### SUMMARY OF THE INVENTION

### TECHNICAL PROBLEM

In view of the above-mentioned deficiencies in the prior art, the present invention provides a long-acting super-hydrophobic anticoagulant biological valve and the preparation method thereof, which can prepare a layer of super-hydrophobic coating with high water contact angle and a low rolling angle on the surface of the biological valve material without changing the mechanical properties of the valve body, thereby improving the antithrombotic performance of the biological valve after being implanted in the human body.

### TECHNICAL SOLUTION

In order to achieve the above object, the technical solution adopted by the present invention to solve the technical problems is:

A preparation method for a long-acting super-hydrophobic anticoagulant biological valve, includes the following steps:
(1) treating a biological valve material with glutaraldehyde;
(2) placing the biological valve material treated in step (1) in an acid solution containing a polyphenol compound and metal ions, and adding an oxidant for reaction, thereby embedding nanoparticles formed by polymerization of a polyphenol compound and metal ions and containing double bond groups in the surface of the biological valve material;
(3) reacting the biological valve material treated in step (2) with a hydrophobic substance, thereby grafting a hydrophobic substance on the surfaces of the nanoparticles to form a hydrophobic coating.

Further, the treating with glutaraldehyde in step (1) specifically includes immersing the biological valve material in a glutaraldehyde solution for 48 h to 96 h.

Further, in step (2), the oxidant has a concentration of 20 µM to 1 mM, and the reaction is performed at 10 °C to 40 °C for 20 min to 120 min.

Further, the molar mass ratio of the polyphenol compound and the metal ions is 1 : 0.01 to 1.

Further, the polyphenol compound is at least one of tannic acid, gallic acid, salvianolic acid B, epigallocatechin gallate, epicatechin gallate, epicatechin, epigallocatechin, catechol, pyrogallol, and flavonoid.

Further, the metal ions are at least one of copper ions, silver ions and iron ions.

Further, the acid solution has a pH value ranging from 4 to 6.

Further, the acid solution is acetic acid - acetate buffer, 2- (N- morpholine) ethanesulfonic acid buffer, glycine - hydrochloric acid buffer, phthalic acid - hydrochloric acid buffer, potassium hydrogen phthalate -sodium hydroxide buffer, disodium hydrogen phosphate - citric acid buffer or citric acid - sodium hydroxide - hydrochloric acid buffer.

Further, the oxidant is a water-soluble oxidant, which is, in particular, at least one of hydrogen peroxide, ammonium persulfate, cupric chloride, ferric chloride, concentrated nitric acid, sodium periodate, potassium permanganate and potassium dichromate.

Further, the step (3) specifically includes:
immersing the biological valve material treated in step (2) in the hydrophobic substance with a concentration of 50 µM to 1 mM for 10 min to 30 min, and forming a hydrophobic coating on the surfaces of the nanoparticles by means of click chemistry.

Further, the hydrophobic substance is perfluoropentadecane, perfluorododecanoic acid, 1H, 1H, 2H, 2H- perfluorododecanethiol, perfluorobutylethylene, stearoyl chloride or stearic acid.

Further, the biological valve material is an aortic valve, a pulmonary valve, a venous valve, a mitral valve or a tricuspid valve.

Further, the biological valve material is selected from porcine pericardium or bovine pericardium or the like.

The present invention further provides a long-acting super-hydrophobic anticoagulant biological valve, including a biological valve material, nanoparticles embedded in a surface of the biological valve material and formed by polymerization of a polyphenol compound and metal ions and containing double bond groups, and a hydrophobic coating formed by grafting a hydrophobic substance on surfaces of the nanoparticles.

The long-acting super-hydrophobic anticoagulant biological valve can be prepared by any of the above-mentioned preparation methods.

### TECHNICAL ADVANTAGES

The technical advantages of the present invention are:
1. In the present invention, a polyphenol compound can be introduced by using the residual amino and aldehyde groups on the surface of the biological valve material through the reactivity of polyphenol-related functional groups. In addition, the nanoparticle product obtained by oxidation of the polyphenol compound and metal in the presence of strong oxidants is rich in double bond groups, and a hydrophobic fluoride with functional group modification can be subsequently introduced through click chemistry to prepare a biological valve material with long-acting super-hydrophobic anticoagulant property. The method of the invention involves a simple and controllable preparation process with mild conditions, the super-hydrophobic performance of the coating is stable, and platelet adhesion and thrombosis on the valve surface can be effectively prevented.
2. Inspired by the mild self-polymerization reaction conditions of polyphenol and the reaction mechanism of metal ions, double bonds and sulfhydryl groups, a super-hydrophobic coating having a long-acting high water contact angle and a low rolling angle is prepared on the surface of the biological valve by means of a simple and stable operation process without affecting the performance of the valve body, and the requirements of long-acting anticoagulation are met by resisting the adsorption of plasma proteins.

### BRIEF DESCRIPTION OF DRAWINGS

FIG. 1 is an SEM image of a biological valve super-hydrophobic fluoride coating prepared according to Example 1;
FIG. 2 shows the contact angle of the coating showing in FIG. 1;
FIG. 3 shows the platelet adhesion of a traditional glutaraldehyde valve in vitro;
FIG. 4 shows the platelet adhesion of a super-hydrophobic modified valve material prepared according to Example 2 of the present invention in vitro.

### DDESCRIPTION OF EMBODIMENTS

The specific embodiments of the present invention are described below so that those skilled in the art can understand the present invention, but it should be noted that the present invention is not limited to the scope of the specific embodiments. For those skilled in the art, various modifications within the spirit and scope of the present invention defined and determined by the appended claims are obvious, and all inventions using the concept of the present invention fall in the protection scope of the present invention.

### Example 1

A method for preparing a long-acting super-hydrophobic anticoagulant biological valve, includes the following steps:
(1) Treat a porcine pericardial valve material cross-linked by glutaraldehyde;
(2) Immerse the porcine pericardial valve material treated in step (1) in a buffer solution containing tannic acid and Cu²⁺ ions at a molar mass ratio of 1 : 0.1. The buffer solution is acetic acid - sodium acetate, and its pH value is 4. Then add an oxidant - sodium periodate with a concentration of 20 µM into the solution, and react at 25 °C for 40 minutes for an oxidative polymerization of the tannic acid and chelation with the Cu²⁺ ions at the same time, thereby forming polyphenol nanoparticles containing Cu²⁺ ions on the surface of the biological valve material;
(3) Immerse the porcine pericardial valve material with polyphenol nanoparticles containing Cu²⁺ on the surface into 1H, 1H, 2H, 2H- perfluorododecanethiol with a molar concentration of 0.8 mM for 20 min, and then generate a uniform fluoride coating on the surface of the valve material by chemical grafting, thereby preparing a biological valve material with a super-hydrophobic coating on the surface.

### Example 2

A method for preparing a long-acting super-hydrophobic anticoagulant biological valve, includes the following steps:
(1) Treat a porcine pericardial valve material cross-linked by glutaraldehyde;
(2) Immerse the porcine pericardial valve material treated in step (1) in a buffer solution containing epigallocatechin gallate and Ag⁺ ions at a molar mass ratio of 1 : 0.05. The buffer solution is glycine - hydrochloric acid buffer solution, and its pH value is 4. Then add an oxidant - concentrated nitric acid with a concentration of 80 µM into the solution, and react at 25 °C for 60 minutes for an oxidative polymerization of the epigallocatechin gallate and chelation with the Ag⁺ ions at the same time, thereby forming polyphenol nanoparticles containing Ag⁺ ions on the surface of the biological valve material;
(3) Immerse the porcine pericardial valve material with polyphenol nanoparticles containing Ag⁺ on the surface into perfluorododecanoic acid with a molar concentration of 0.5 mM for 25 min, and then generate a uniform fluoride coating on the surface of the valve material by chemical grafting, thereby preparing a biological valve material with a super-hydrophobic coating on the surface.

### Example 3

A method for preparing a long-acting super-hydrophobic anticoagulant biological valve, includes the following steps:
(1) Treat a bovine pericardial valve material cross-linked by glutaraldehyde;
(2) Immerse the porcine pericardial valve material treated in step (1) in a buffer solution containing epicatechin and Ag⁺ ions at a molar mass ratio of 1 : 0.2. The buffer solution is disodium hydrogen phosphate - citric acid, and its pH value is 4. Then add an oxidant - potassium permanganate with a concentration of 0.05 mM into the solution, and react at 20 °C for 40 minutes for an oxidative polymerization of the epicatechin and chelation with the Ag⁺ ions at the same time, thereby forming polyphenol nanoparticles containing Ag⁺ ions on the surface of the biological valve material;
(3) Immerse the bovine pericardial valve material with polyphenol nanoparticles containing Ag⁺ on the surface into stearoyl chloride with a molar concentration of 0.2 mM for 10 min, and then generate a uniform super-hydrophobic coating on the surface of the valve material by chemical grafting, thereby preparing a biological valve material with a super-hydrophobic coating on the surface.

### Example 4

A method for preparing a long-acting super-hydrophobic anticoagulant biological valve, includes the following steps:
(1) Treat a bovine pericardial valve material cross-linked by glutaraldehyde;
(2) Immerse the porcine pericardial valve material treated in step (1) in a buffer solution containing pyrogallol and Fe³⁺ ions at a molar mass ratio of 1 : 0.05. The buffer solution is citric acid - sodium hydroxide - hydrochloric acid buffer solution, and its pH value is 4. Then add an oxidant - ammonium persulfate with a concentration of 0.01 mM into the solution, and react at 20 °C for 60 minutes for an oxidative polymerization of the pyrogallol and chelation with the Fe³⁺ ions at the same time, thereby forming polyphenol nanoparticles containing Fe³⁺ ions on the surface of the biological valve material;
(3) Immerse the bovine pericardial valve material with polyphenol nanoparticles containing Fe³⁺ on the surface into stearic acid with a molar concentration of 0.05 mM for 30 min, and then generate a uniform fluoride coating on the surface of the valve material by chemical grafting, thereby preparing a biological valve material with a super-hydrophobic coating on the surface.

### Experimental example

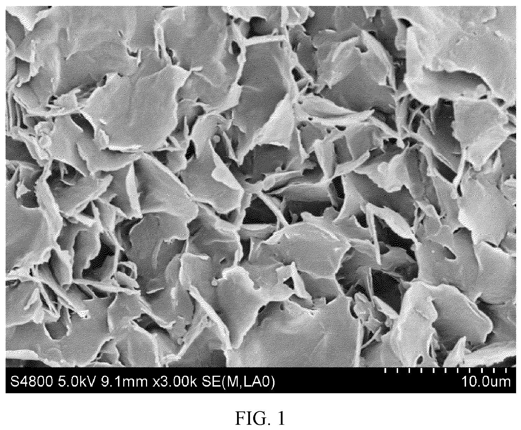

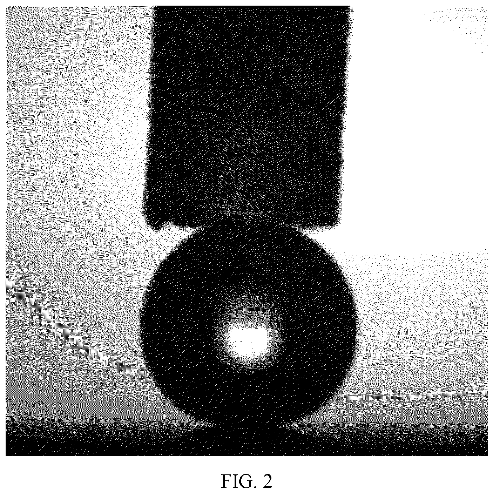

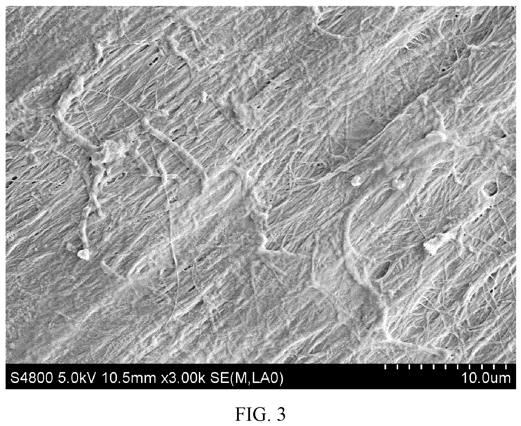

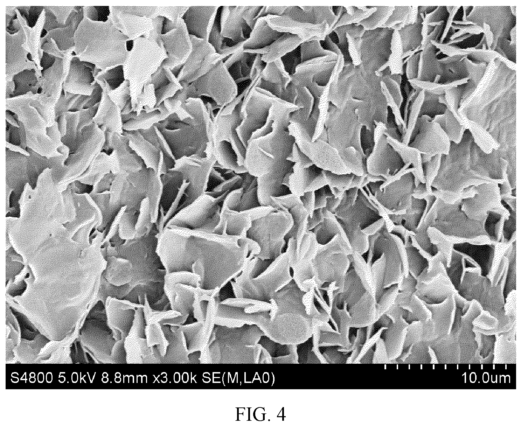

1. The SEM image of the super-hydrophobic coating of the biological valve material prepared in Example 2 is shown in FIG. 1, and the test result of the contact angle is shown in FIG. 2.
   As shown in FIGS. 1 and 2, the hydrophobic coating prepared by the present application is evenly distributed on the surface of the valve material, and has long-term high water contact angle and low rolling angle.
2. Taking a glutaraldehyde valve as a control group and the biological valve material prepared in Example 2 as the experimental group, an in vitro platelet adhesion experiment was carried out to detect the anticoagulant performance, and the results are shown in FIG. 3 and FIG. 4.
   As shown in FIG. 3 and FIG. 4, compared with the glutaraldehyde valve, the biological valve material prepared by the present application has excellent anticoagulant effect.

## Claims

1. A preparation method for a long-acting super-hydrophobic anticoagulant biological valve, comprising the following steps:
(1) treating a biological valve material with glutaraldehyde;
(2) placing the biological valve material treated in step (1) in an acid solution containing a polyphenol compound and metal ions, and adding an oxidant for reaction; and
(3) reacting the biological valve material treated in step (2) with a hydrophobic substance.

2. The preparation method for the long-acting super-hydrophobic anticoagulant biological valve according to claim 1, wherein the treating with glutaraldehyde in step (1) includes immersing the biological valve material in a glutaraldehyde solution for 48 h to 96 h.

3. The preparation method for the long-acting super-hydrophobic anticoagulant biological valve according to claim 1, wherein in step (2), the oxidant has a concentration of 20 µM to 1 mM, and the reaction is performed at 10 °C to 40 °C for 20 min to 120 min.

4. The preparation method for the long-acting super-hydrophobic anticoagulant biological valve according to claim 1, wherein a molar mass ratio of the polyphenol compound and the metal ions is 1 : 0.01 to 1.

5. The preparation method for the long-acting super-hydrophobic anticoagulant biological valve according to claim 4, wherein the polyphenol compound is at least one of tannic acid, gallic acid, salvianolic acid B, epigallocatechin gallate, epicatechin gallate, epicatechin, epigallocatechin, catechol, pyrogallol, and flavonoid.

6. The preparation method for the long-acting super-hydrophobic anticoagulant biological valve according to claim 4, wherein the metal ions are at least one of copper ions, silver ions and iron ions.

7. The preparation method for the long-acting super-hydrophobic anticoagulant biological valve according to claim 1, wherein the acid solution has a pH value ranging from 4 to 6.

8. The preparation method for the long-acting super-hydrophobic anticoagulant biological valve according to claim 1, wherein the acid solution is acetic acid - acetate buffer, 2- (N-morpholine) ethanesulfonic acid buffer, glycine - hydrochloric acid buffer, phthalic acid - hydrochloric acid buffer, potassium hydrogen phthalate -sodium hydroxide buffer, disodium hydrogen phosphate - citric acid buffer or citric acid - sodium hydroxide - hydrochloric acid buffer.

9. The preparation method for the long-acting super-hydrophobic anticoagulant biological valve according to claim 1, wherein the oxidant is a water-soluble oxidant, which is, in particular, at least one of hydrogen peroxide, ammonium persulfate, cupric chloride, ferric chloride, concentrated nitric acid, sodium periodate, potassium permanganate and potassium dichromate.

10. The preparation method for the long-acting super-hydrophobic anticoagulant biological valve according to claim 1, wherein step (3) includes:
immersing the biological valve material treated in step (2) in the hydrophobic substance with a concentration of 50 µM to 1 mM for 10 min to 30 min.

11. The preparation method for the long-acting super-hydrophobic anticoagulant biological valve according to claim 10, wherein the hydrophobic substance is perfluoropentadecane, perfluorododecanoic acid, 1H, 1H, 2H, 2H- perfluorododecanethiol, perfluorobutylethylene, stearoyl chloride or stearic acid.

12. The preparation method for the long-acting super-hydrophobic anticoagulant biological valve according to claim 1, wherein the biological valve material is an aortic valve, a pulmonary valve, a venous valve, a mitral valve or a tricuspid valve.

13. The preparation method for the long-acting super-hydrophobic anticoagulant biological valve according to claim 12, wherein the biological valve material is selected from porcine pericardium or bovine pericardium.

14. A long-acting super-hydrophobic anticoagulant biological valve, comprising a biological valve material, nanoparticles embedded in a surface of the biological valve material and formed by polymerization of a polyphenol compound and metal ions and containing double bond groups, and a hydrophobic coating formed by grafting a hydrophobic substance on surfaces of the nanoparticles.
